(19) Europäisches Patentamt
European Patent Office
Office européen des brevets

(11) EP 0 672 674 B1

(12) EUROPÄISCHE PATENTSCHRIFT

(45) Veröffentlichungstag und Bekanntmachung des Hinweises auf die Patenterteilung:
**24.05.2000 Patentblatt 2000/21**

(21) Anmeldenummer: **95108100.9**

(22) Anmeldetag: **24.11.1989**

(51) Int Cl.[7]: **C07F 13/00**, B01J 31/16, C07F 15/00, C07F 9/50, C07C 5/03, C07C 45/50, C07C 45/30, C07D 301/00, C07F 9/53, C07C 2/86, C07F 15/02

(54) **Sulfonierte Phenylphosphane enthaltende Komplexverbindungen**

Sulfonated phenyl phosphine-containing complexes

Complexes contenant de la phényl phosphine sulfonée

(84) Benannte Vertragsstaaten:
**AT DE ES FR GB SE**

(30) Priorität: **02.12.1988 DE 3840600**
**29.06.1989 DE 3921295**

(43) Veröffentlichungstag der Anmeldung:
**20.09.1995 Patentblatt 1995/38**

(62) Dokumentnummer(n) der früheren Anmeldung(en) nach Art. 76 EPÜ:
**89121708.5 / 0 372 313**

(73) Patentinhaber: **Celanese Chemicals Europe GmbH**
**60439 Frankfurt am Main (DE)**

(72) Erfinder:
- **Herrmann, Wolfgang, Prof. Dr.**
**D-85354 Freising (DE)**
- **Kulpe, Jürgen**
**D-65929 Frankfurt (DE)**
- **Kellner, Jürgen**
**D-85716 Unterschleissheim (DE)**
- **Riepl, Herbert**
**D-85221 Dachau (DE)**

(56) Entgegenhaltungen:
**EP-A- 0 133 410**
**DE-A- 2 700 904**
**GB-A- 1 095 146**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft neue Komplexverbindungen von Elementen der Gruppe VII A des Periodensystems. Gemeinsames Merkmal dieser Verbindungen ist, daß sie als Komplexligand das Trinatriumsalz von Tris(m-sulfophenyl) phosphan und gegebenenfalls weitere Liganden enthalten. Die Verbindungen sind in Wasser ohne Zersetzung löslich.

Komplexverbindungen definierter Zusammensetzung, die das Trinatriumsalz des m-trisulfonierten Triphenylphosphans der chemischen Formel $P(C_6H_4\text{-m-}SO_3Na)_3$ als einzigen oder als einen von mehreren Liganden enthalten, sind kaum bekannt. In der DE 27 00 904 C2, Beispiel 12, wird die Umsetzung von Bis(1,5-cyclooctadien)nickel mit dem Trinatriumsalz von Tris(m-sulfophenyl)phosphan (im folgenden TPPTS genannt) beschrieben. Man erhält eine rotgefärbte Verbindung, die aus ihrer wäßrigen Lösung durch Eindampfen im Vakuum als feste Substanz gewonnen wird. Nach Angaben der Autoren soll es sich bei dieser Verbindung um das Tetrakis-trinatriumsalz von Tris[(m-sulfophenyl)phosphan]nickel(O) handeln. In der gleichen Druckschrift finden sich auch pauschale Angaben über die Herstellung von TPPTS-Komplexverbindungen des Eisens und des Palladiums. Demnach soll man wasserlösliche Verbindungen oder solche Verbindungen, die unter Reaktionsbedingungen in Lösung gehen, in Gegenwart eines Reduktionsmittels mit wäßriger TPPTS-Lösung umsetzen. Als Reduktionsmittel kommen z.B. $Na[BH_4]$, $K[BH_4]$, Zinkpulver, Magnesium oder Borhydride in Betracht. Weder das Herstellungsverfahren noch einzelne Verbindungen werden durch Beispiele näher beschrieben oder gar charakterisiert.

Komplexverbindungen, die TPPTS als Liganden enthalten, ohne daß die genaue Zusammensetzung dieser Verbindungen bekannt ist, bilden sich bei verschiedenen Umsetzungen aus Metall oder Metallverbindungen, TPPTS und gegebenenfalls weiteren Liganden. So haben speziell Rhodium-Komplexe mit TPPTS-Liganden in jüngster Zeit als Bestandteil von Katalysatorsystemen Bedeutung erlangt, die bei der Hydroformylierung von Olefinen Anwendung finden. Gegenüber anderen Katalysatoren, die für dieselbe Reaktion eingesetzt werden, haben sie den Vorteil, in Wasser löslich zu sein. Daher kann die Hydroformylierung in einem aus wäßriger und organischer Phase bestehenden heterogenen Reaktionsmedium (Zweiphasensystem) durchgeführt werden mit dem Ergebnis, daß sich das Reaktionsprodukt durch einfache Phasentrennung vom wasserlöslichen Katalysator abtrennen läßt. Darüberhinaus stellt diese Arbeitsweise sicher, daß der wertvolle Edelmetallkatalysator annähernd verlustfrei wiedergewonnen oder in die Synthesestufe zurückgeführt werden kann. Ein derartiger Prozeß ist z.B. in der DE 26 27 354 B2 beschrieben.

Auch die Anlagerung von Cyanwasserstoff an ungesättigte organische Verbindungen kann in Gegenwart einer Verbindung des nullwertigen Nickels oder des Eisens oder Palladiums in reduzierter Wertigkeitsstufe und einer wäßrigen Lösung eines sulfonierten Triphenylphosphans, insbesondere einer wäßrigen Lösung von TPPTS, als Katalysator erfolgen. Diese Arbeitsweise ist in der bereits zitierten DE 27 00 904 C2 beschrieben. Statt der Komponenten Nickelsalz und TPPTS-Lösung kann auch eine eigens hergestellte Komplexverbindung, der die Zusammensetzung $Ni(TPPTS)_4$ zugeschrieben wird, als Katalysator eingesetzt werden.

Trotz der geschilderten Vorteile der Verwendung wasserlöslicher TPPTS-Komplexverbindungen als Katalysatoren ist über ihren Einsatz bei anderen Reaktionen nichts bekannt geworden. Dieser Umstand ist wohl in erheblichem Maße darauf zurückzuführen, daß es trotz intensiver Bemühungen bisher nicht gelang, TPPTS enthaltende, wasserlösliche Komplexverbindungen in reiner Form zu isolieren und damit die Voraussetzung für ihre stoffliche Charakterisierung durch chemische und physikalische Analysenverfahren zu schaffen.

Der vorliegenden Erfindung liegt daher die Aufgabe zugrunde, TPPTS enthaltende Komplexverbindungen bestimmter Metalle in definierter, reproduzierbarer Zusammensetzung bereitzustellen.

Die Erfindung besteht in neuen Komplexverbindungen von Elementen der Gruppe VII A des Periodensystems. Die Verbindungen sind dadurch gekennzeichnet, daß sie als Komplexligand das Trinatriumsalz von Tris(m-sulfophenyl) phosphan und gegebenenfalls weitere Liganden enthalten.

Die neuen Verbindungen können durch die allgemeine Formel

$$L^1_w L^2_x M_y (TPPTS)_z$$

wiedergegeben werden. In dieser Formel bedeuten $L^1$ und $L^2$ gleiche oder unterschiedliche Liganden, die in der Komplexverbindung neben TPPTS an das Zentralatom gebunden sein können, wie z.B. H, CO, NO, $PF_3$, $H_2O$, S, Halogen wie Cl, ferner $\pi$-Aromatenliganden wie Cyclopentadienyl, $\pi$-Olefinliganden wie Cyclooctadien oder $\pi$-Acetylenliganden wie Diphenylacetylen; M steht für die Elemente der Gruppe VII A des Periodensystems als Zentralatom, insbesondere für Mangan; w, x, y und z sind ganze Zahlen, wobei w und x jeweils 0 bis 7y bedeuten, y 1 bis 6 und $z \leq 4y$ sind.

Die neuen Verbindungen sind kristalline, zumeist farbige Substanzen. Sie sind in Wasser ohne Zersetzung löslich und können aus der wäßrigen Lösung als Hydrate in Form von Pulvern oder Kristallen isoliert werden. Diese Hydrate enthalten je Natriumion ein Molekül Wasser. Bei Raumtemperatur sind sie mehrheitlich an der Luft beständig.

Die beanspruchten Verbindungen sind präparativ auf verschiedenen Wegen zugänglich:

- durch Synthese aus einfachen Verbindungen, d.h. Salzen jenes Elements, das das Zentralatom der Komplexverbindung bildet;

- durch Ligandenaustauschreaktion aus Komplexverbindungen gemäß

$$L^1_w L^2_{x+z} M_y + z\ TPPTS \rightarrow L^1_w L^2_x M_y (TPPTS)_z + zL^2$$

  wobei $L^1$, $L^2$ und M sowie x, y und z die obengenannten Bedeutungen haben, mit der Maßgabe, daß z kleiner oder gleich x ist;

- durch Einführung von TPPTS-Liganden in Komplexverbindungen, die nicht durch einfachen Ligandenaustausch im Sinne der vorstehenden Gleichung, sondern durch Eliminierungs- und/oder Substitutionsreaktionen erfolgt.

Zur Synthese aus einfachen Verbindungen geht man zweckmäßig von wasserlöslichen Salzen aus. Die Art des Anions hat im allgemeinen keinen Einfluß auf den Ablauf der Reaktion. Geeignet sind z.B. die Halogenide, insbesondere die Chloride, Salze von Sauerstoffsäuren wie Nitrate und Sulfate sowie Salze von Carbonsäuren wie Formiate und Acetate. Das in Wasser gelöste Salz wird mit einer wäßrigen TPPTS-Lösung im stöchiometrischen Verhältnis oder im Überschuß umgesetzt. Falls die Oxidationsstufe des Metalls in der Komplexverbindung niedriger als in dem Ausgangssalz ist, kann entweder überschüssiges TPPTS als Reduktionsmittel wirken oder man setzt der Reaktionslösung ein Reduktionsmittel zu. Geeignet sind z.B. $Na[BH_4]$ oder Hydrazinhydrat. In diesem Fall empfiehlt es sich auch unter Luftausschluß zu arbeiten. Im allgemeinen läuft die Umsetzung bereits bei Raumtemperatur ab; nur selten muß sie durch Erhöhung der Temperatur beschleunigt oder vervollständigt werden.

Bei der Herstellung durch Ligandenaustausch setzt man als Ausgangssubstanzen Komplexverbindungen der jeweiligen Metalle ein. Entsprechend ihrer Löslichkeit werden sie in Wasser oder in einem organischen Lösungsmittel wie aromatischen Kohlenwasserstoffen (z.B. Toluol), Halogenkohlenwasserstoffen (z.B. Chloroform), Alkoholen (z.B. Ethanol) oder heterocyclischen Verbindungen (z.B. Tetrahydrofuran) gelöst. TPPTS wird wiederum in Form einer wäßrigen Lösung verwendet und im stöchiometrischen Verhältnis oder im Überschuß eingesetzt.

Auch im Falle der Synthese durch Ligandenaustausch genügt es, bei Raumtemperatur zu arbeiten. Selbst wenn die Ausgangskomplexverbindung in einem mit Wasser nicht mischbaren organischen Lösungsmittel gelöst ist, die Umsetzung also in einem flüssigen Zweiphasensystem abläuft, reichen kurze Reaktionszeiten aus; intensive Rührung fördert hierbei die Reaktion.

Die Herstellung der neuen Komplexverbindungen durch Eliminierungs- und Substitutionsreaktionen erfolgt in ähnlicher Weise und unter vergleichbaren Bedingungen wie bei der Synthese durch Ligandenaustausch. Weitere Liganden führt man in die Komplexverbindungen in bekannter Weise ein, z.B. durch Einleiten von CO oder durch Zugabe einer den Nitrosylrest, eine Schwefel- oder Hydridgruppe abspaltenden Verbindung.

Zur Aufarbeitung des Reaktionsproduktes und zur Isolierung der neuen Verbindungen, die unabhängig vom Herstellungsverfahren, in wäßriger Lösung vorliegen, dampft man das Wasser, gegebenenfalls nach vorheriger Filtration der Lösung, im Vakuum ab. Im allgemeinen erhält man auf diesem Wege nicht die reinen Verbindungen, sondern verunreinigte Produkte oder auch Gemische verschiedener TPPTS-Komplexverbindungen, die sich bei der Herstellung nebeneinander gebildet haben. Es ist daher erforderlich, zur Gewinnung der Reinsubstanzen spezielle Reinigungs- und Trennverfahren anzuwenden. Als besonders geeignet zur Lösung dieser Aufgabe hat sich die Chromatographie an Gelen erwiesen, die Gegenstand der deutschen Patentanmeldung P 38 22 036.9 ist. Angepaßt an die Eigenschaften der jeweiligen Verbindung, muß hierbei gegebenenfalls auf Licht- oder Luftausschluß geachtet werden; auch die Elutionsmittel und -geschwindigkeiten richten sich nach dem jeweiligen Reinigungs- bzw. Trennproblem. Nach dieser Behandlung liegen die Verbindungen analysenrein und spektroskopisch rein vor.

Wie bereits weiter oben gesagt wurde, kristallisieren die neuen Verbindungen aus wäßriger Lösung als Hydrate. Durch Entwässerung unter schonenden Bedingungen, d.h. bei Temperaturen unterhalb des Schmelz- bzw. Zersetzungspunktes und unter Anwendung von vermindertem Druck, zweckmäßig Hochvakuum, lassen sich aus ihnen, ohne daß Zersetzung eintritt, die wasserfreien Verbindungen herstellen. Der beanspruchte Schutz erstreckt sich daher sowohl auf die wasserhaltigen als auch auf die wasserfreien TPPTS-Komplexe.

Die erfindungsgemäßen Verbindungen sind katalytisch aktiv und werden mit Erfolg als Katalysatoren oder Bestandteile von Katalysatoren bei verschiedenen Reaktionen eingesetzt.

Besonders hervorzuheben ist, daß durch Einsatz der reinen Verbindungen ausbeutemindernde Neben- und Folgereaktionen vermieden werden, die häufig dann auftreten, wenn der Katalysator im Reaktionsgemisch "in situ" gebildet wird. Dieser Sachverhalt beruht darauf, daß die "in situ-Herstellung" im allgemeinen mit der Bildung unwirksamer oder störender Nebenprodukte verbunden ist. In den von anderen Autoren beschriebenen Fällen enthalten solche Katalysatoren ausnahmslos noch freies, überschüssiges TPPTS, welches die Reaktivität der eigentlichen TPPTS-Komplexverbindung stark verändert. Die Verwendung reiner TPPTS-Komplexverbindungen hat gezeigt, daß diese Verbindungsklasse in viel umfangreicherem Maße katalytisch wirksam ist, als bisher bekannt und zu erwarten war.

So sind die erfindungsgemäßen Komplexverbindungen ausgezeichnete Hydrierkatalysatoren. Beispielsweise werden sie mit Erfolg für die Hydrierung von Olefinen zu gesättigten Kohlenwasserstoffen eingesetzt. Die Reaktion verläuft in ihrer Gegenwart schon bei Normaldruck und Temperaturen zwischen 20 und 40 °C.

Auch die Verschiebung des Wassergasgleichgewichts

$$CO + H_2O \rightleftharpoons CO_2 + H_2$$

in Richtung auf Wasserstoff- und Kohlendioxidbildung wird durch die neuen Verbindungen bewirkt. Sie ermöglichen es, die Umsetzung bereits bei Raumtemperatur und etwa 1,5 MPa Druck durchzuführen. Auf diesem Wege gebildeter Wasserstoff kann für katalytische Hydrierungen, z.B. die Umsetzung organischer Nitroverbindungen zu organischen Aminen, genutzt werden. Da bei der obigen Reaktion neben Wasserstoff immer Kohlenmonoxid vorhanden ist, kann das Gasgemisch auch zur Hydroformylierung von Olefinen verwendet werden.

Die Wasserstofferzeugung aus Wasser und Kohlenmonoxid in Gegenwart der erfindungsgemäßen Verbindungen als Katalysatoren ermöglicht auch die Hydrocarbonylierung von Olefinen. Besonders bemerkenswert ist, daß die Reaktion schon bei relativ niedrigen Temperaturen abläuft. So erhält man z.B. aus Ethylen bei 140 bis 150 °C nach der Gleichung

$$H_2C{=}CH_2 + CO + H_2 \rightarrow H_5C_2 - CO - C_2H_5$$

Diethylketon.

Weiterhin können die neuen Komplexverbindungen als Katalysatoren für die Hydroformylierung olefinisch ungesättigter Verbindung verwendet werden. Sie haben sich sowohl bei der Umsetzung linearer und cyclischer Olefine als auch bei der Umsetzung von Verbindungen bewährt, die neben einer Doppelbindung auch funktionelle Gruppen im Molekül enthalten.

Auch die Oxidation unterschiedlicher Verbindungsklassen wird durch die neuen, TPPTS als Komplexligand enthaltenden Verbindungen katalysiert. So erhält man mit Iodosobenzol als Oxidationsmittel aus sekundären Alkoholen die entsprechenden Ketone, aus Olefinen Epoxide und aus Alkinen Diketone.

Die erfindungsgemäßen Komplexverbindungen katalysieren darüber hinaus Reaktionen, bei denen Kohlenstoff-Kohlenstoff-Bindungen neu gebildet werden. Ein Beispiel für diesen Reaktionstyp ist die Allen-Alkin-Kupplung gemäß

$$R^1R^2C{=}C{=}CHX + HC{\equiv}CR^3 \rightarrow R^1R^2C{=}C{=}CH\text{-}C{\equiv}CR^3 + HX$$

wobei $R^1$, $R^2$, $R^3$ Alkyl- und/oder Arylgruppen, $R^1$ auch H, sind. Exemplarisch für diese Umsetzung ist die Reaktion von 1-Bromallen mit Phenylacetylen, die unter der katalytischen Einwirkung der neuen Verbindungen schon bei Raumtemperatur vor sich geht.

Schließlich katalysieren die erfindungsgemäßen Komplexverbindungen die Addition von sekundären organischen Aminen an Kohlenstoff-Kohlenstoff-Doppelbindungen nach dem Schema

$$R^1R^2C{=}CH_2 + HNR^3 \rightarrow R^1R^2CH\text{-}CH_2\text{-}NR^3$$

Die Substituenten $R^1$, $R^2$ und $R^3$ haben die oben wiedergegebene Bedeutung.

<u>Beispiel</u>:

TPPTS wurde nach dem in der DE 32 35 030 Al beschriebenen Verfahren hergestellt und gereinigt. $OP(C_6H_4\text{-}m\text{-}SO_3Na)_3$, im folgenden TPPOTS genannt, wird durch Gelchromatographie nach dem in der deutschen Patentanmeldung DE-A-38 22 036 beschriebenen Verfahren abgetrennt. Die angegebenen Ausbeuten beziehen sich auf die gereinigten Substanzen.

Bei den zur Charakterisierung der neuen Substanzen herangezogenen NMR- und IR-Daten wurden folgende

Abkürzungen verwendet:
s = Singulett, d = Dublett, t = Triplett,
m = Multiplett
ss = sehr schwach, s = schwach, m = mittelstark,
st = stark, sst = sehr stark, b = breite Bandenform,
Sch = Schulter.

Die zur chromatographischen Reinigung der neuen Substanzen verwendeten Sephadex-Gele sind mit Epichlorhydrin vernetzte Dextrane. Bei Fractogel handelt es sich um Oligoethylglykol-Glycydyl/Methacrylat/Pentaerythroldimethacrylat-Copolymerisat.

Synthese von ($\eta^5$-$C_5H_5$)Mn$(CO)_2$(TPPTS) · 3 $H_2O$ und ($\eta^5$-$C_5H_5$)Mn(CO)$(TPPTS)_2$ · 6 $H_2O$

1.05 g (5 mmol) ($\eta^5$-$C_5H_5$)Mn$(CO)_3$ ("Cymantren") werden in 70 ml Tetrahydrofuran gelöst. Die gelbe Lösung bestrahlt man in einer Tauchlampen-Bestrahlungsapparatur aus Duranglas (wassergekühlter Quecksilber-Hochdruckbrenner TQ 150 der Original Quarzlampen Gesellschaft mbH, Hanau) 90 min bei 15°C. Die karminrote Lösung wird sodann zu einer Lösung von 1.42 g (2.5 mmol) TPPTS in 10 ml Wasser gegeben. Man läßt 16 h rühren, wobei sich die organische Phase entfärbt und die wäßrige Phase orange färbt. Nach Trennung der Phasen wäscht man die wäßrige Phase zweimal mit je 25 ml n-Pentan und dampft darauf das Wasser im Vakuum ab.

Die beiden im Rückstand enthaltenen Verbindungen werden durch Säulenchromatographie an Sephadex G-15 getrennt. Die erste, gelborange Zone enthält den hydratisierten Diphosphan-Komplex($\eta^5$-$C_5H_5$)Mn(CO)$(TPPTS)_2$, die zweite, gelbe Zone den hydratisierten Monophosphan-Komplex ($\eta^5$-$C_5H_5$)Mn$(CO)_2$(TPPTS) neben freiem TPPTS.

Charakterisierung ($\eta^5$-$C_5H_5$)Mn(CO)$(TPPTS)_2$ · 6 $H_2O$

$^{31}$P-NMR (109.3 MHz, $D_2O$, 5 °C) : $\delta$ = 94.9 ppm (s).
$^1$H-NMR (270 MHz, $D_2O$, 5 °C) : $\delta$ = 3.97 ppm [S,$C_5H_5$, 5H]; = 7.11-7.99 ppm [m,$C_6H_4$, 24H].
IR (cm $^{-1}$, KBr): ν(CO) = 1828 (st); ν(SO) = 1221 (Sch, sst), 1197 (sst), 1039 (sst), 624 (sst).

Elementaranalyse ($C_{42}H_{41}MnNa_6O_{25}P_2$; 1200,61)
Ber. C 36.22 H 2.97 Mn 3.94 O 28.71 P 4.45 S 13.81
Gef. C 36.15 H 2.68 Mn 3.85 O 27.14 P 4.67 S 14.00

Charakterisierung ($\eta^5$-$C_5H_5$)Mn$(CO)_2$(TPPTS) · 3 $H_2O$

$^{31}$P-NMR (109.3 MHz, $D_2O$, 5 °C) : $\delta$ = 95.7 (s).
$^1$H-NMR (270 MHz, $D_2O$, 5 °C) : $\delta$ = 4.36 [s, $C_5H_5$, 5H], $\delta$ = 7.37-8.07 [m, $C_6$ $H_4$ , 12H].
IR (cm$^{-1}$, KBr) : ν(CO) = 1929 (sst), 1852 (sst); ν(SO) = 1224 (Sch, sst), 1199 (sst), 1040 (sst), 622 (sst).

**Patentansprüche**

1. Komplexverbindungen von Elementen der Gruppe VII A des Periodensystems, dadurch gekennzeichnet, daß sie als Komplexligand das Trinatriumsalz von Tris(m-sulfophenyl)phosphan und gegebenenfalls weitere Liganden enthalten.

2. Komplexverbindungen nach Anspruch 1, dadurch gekennzeichnet, daß sie der allgemeinen Formel

$$L^1_w L^2_x M_y[P(C_6H_4\text{-m-}SO_3Na)_3]_z$$

folgen, wobei $L^1$ und $L^2$ gleiche oder unterschiedliche Liganden, die neben dem Trinatriumsalz von Tris(m-sulfophenyl)phosphan an das Zentralatom gebunden sein können, bedeuten, M für die Elemente der Gruppe VII A des Periodensystems als Zentralatom steht, w, x, y und z ganze Zahlen sind, wobei w und x jeweils 0 bis 7y und y 1 bis 6 bedeuten und z ≤4y ist.

3. Komplexverbindungen nach Anspruch 2, dadurch gekennzeichnet, daß sie neben dem Trinatriumsalz von Tris(m-sulfophenyl)phosphan als weitere Liganden H, CO, NO, $PF_3$, S, Halogen, π-Aromaten-Liganden, π-Olefin-Liganden und/oder π-Acetylen-Liganden enthalten.

4. Komplexverbindungen nach Anspruch 3, dadurch gekennzeichnet, daß der π-Aromaten-Ligand Cyclopentadienyl

ist.

**5.** Komplexverbindungen nach Anspruch 3, dadurch gekennzeichnet, daß der π-Olefin-Ligand Cyclooctadien ist.

**6.** Komplexverbindungen nach Anspruch 3, dadurch gekennzeichnet, daß der π-Acetylen-Ligand Diphenylacetylen ist.

**7.** Komplexverbindungen nach Anspruch 1 oder 2, dadurch gekennzeichnet, daß das Zentralatom Mangan, ist.

**8.** Verbindungen nach Anspruch 1 oder 2, dadurch gekennzeichnet, daß sie Manganverbindungen $(\eta^5\text{-}C_5H_5)Mn(CO)_2[P(C_6H_4\text{-m-}SO_3Na)_3]$, $(\eta^5\text{-}C_5H_5)Mn(CO)(P(C_6H_4\text{-m-}SO_3Na)_3]_2$ darstellen.

**9.** Verwendung der Verbindungen nach einem oder mehreren der Ansprüche 1 bis 8 als Hydrierkatalysatoren.

**10.** Verwendung der Verbindungen nach einem oder mehreren der Ansprüche 1 bis 8 als Katalysatoren für die Einstellung des Wassergasgleichgewichts.

**11.** Verwendung der Verbindungen nach einem oder mehreren der Ansprüche 1 bis 8 als Hydrocarbonylierungskatalysatoren.

**12.** Verwendung der Verbindungen nach einem oder mehreren der Ansprüche 1 bis 8 als Hydroformylierungskatalysatoren.

**13.** Verwendung der Verbindungen nach einem oder mehreren der Ansprüche 1 bis 8 als Oxidationskatalysatoren.

**14.** Verwendung der Verbindungen nach einem oder mehreren der Ansprüche 1 bis 8 als Katalysatoren für die Verknüpfung von C-C-Doppelbindungen mit C-C-Mehrfachbindungen.

**15.** Ausführungsform nach Anspruch 14 dadurch gekennzeichnet, daß die Verbindungen als Katalysatoren für die Allen/Alkin-Kupplung verwendet werden.

**16.** Verwendung der Verbindungen nach einem oder mehreren der Ansprüche 1 bis 8 als Katalysatoren für die Addition von sekundären Aminen an C-C-Doppelbindungen.

## Claims

**1.** Complexes of elements of group VII A of the periodic table, characterised in that they contain as complex ligand the trisodium salt of tris(m-sulfophenyl)phosphane and optionally other ligands.

**2.** Complexes according to claim 1, characterised in that they have the formula

$$L^1_w L^2_x M_y [P(C_6H_4\text{-m-}SO_3Na)_3]_z$$

where $L^1$ and $L^2$ are the same or different ligands which apart from the trisodium salt of tris(m-sulfophenyl)phosphane can be bound to the central atom, M stands for elements of group VII A of the periodic table as central atom, w, x, y and z are integers, w and x each being 0 to 7y, y being 1 to 6 and z being ≤ 4y.

**3.** Complexes according to claim 2, characterised in that they contain in addition to the trisodium salt of tris(m-sulfophenyl)phosphane as further ligands H, CO, NO, $PF_3$, S, halogen, π-aromatic ligands, π-olefin ligands and/or π-acetylene ligands.

**4.** Complexes according to claim 3, characterised in that the π-aromatic ligand is cyclopentadienyl.

**5.** Complexes according to claim 3, characterised in that the π-olefin ligand is cyclooctadiene.

6. Complexes according to claim 3, characterised in that the π-acetylene ligand is diphenylacetylene.

7. Complexes according to claim 1 or 2, characterised in that the central atom is manganese.

8. Compounds according to claim 1 or 2, characterised in that they represent manganese compounds $(\eta^5\text{-}C_5H_5)Mn(CO)_2[P(C_6H_4\text{-m-}SO_3Na)_3]$, $(\eta^5\text{-}C_5H_5)Mn(CO)[P(C_6H_4\text{-m-}SO_3Na)_3]_2$.

9. The use of compounds according to one or more of claims 1 to 8 as hydrogenation catalysts.

10. The use of compounds according to one or more of claims 1 to 8 as catalysts to establish the water gas equilibrium.

11. The use of compounds according to one or more of claims 1 to 8 as hydrocarbonylation catalysts.

12. The use of compounds according to one or more of claims 1 to 8 as hydroformylation catalysts.

13. The use of compounds according to one or more of claims 1 to 8 as oxidation catalysts.

14. The use of compounds according to one or more of claims 1 to 8 as catalysts for the linking of C-C double bonds with C-C multiple bonds.

15. Embodiment according to claim 14, characterised in that the compounds are used as catalysts for the allene/alkyne coupling.

16. The use of compounds according to one or more of claims 1 to 8 as catalysts for the addition of secondary amines to C-C double bonds.

**Revendications**

1. Complexes d'éléments du groupe VIIA de la classification périodique, caractérisés en ce qu'ils contiennent en tant que ligand des complexes le sel trisodique du tris(m-sulfophényl)phosphane avec, le cas échéant, d'autres ligands.

2. Complexes selon la revendication 1, caractérisés en ce qu'ils répondent à la formule générale

$$L^1_w L^2_x M_y [P(C_6H_4\text{-m-}SO_3Na)_3]_z$$

dans laquelle $L^1$ et $L^2$ représentent des ligands identiques ou différents qui peuvent être fixés, avec le sel trisodique du tris(m-sulfophényl)phosphane, sur l'atome central, M représente les éléments du groupe VIIA de la classification périodique, qui constituent l'atome central w, x, y et z sont des nombres entiers, w et x ont chacun une valeur de 0 à 7y, y a une valeur de 1 à 6 et $z \leq 4y$.

3. Complexes selon la revendication 2, caractérisés en ce qu'ils contiennent en tant qu'autres ligands, en plus du sel trisodique du tris(m-sulfophényl)phosphane, H, CO, NO, $PF_3$, S, un halogène, des ligands π-aromatiques, des ligands π-oléfiniques et/ou des ligands π-acétyléniques.

4. Complexes selon la revendication 3, caractérisés en ce que le ligand π-aromatique est le cyclopentadiényle.

5. Complexes selon la revendication 3, caractérisés en ce que le ligand π-oléfinique est le cyclooctadiène.

6. Complexes selon la revendication 3, caractérisés en ce que le ligand π-acétylénique est le diphénylacétylène.

7. Complexes selon la revendication 1 ou 2, caractérisés en ce que l'atome central est un atome de manganèse.

8. Composés selon la revendication 1 ou 2, caractérisés en ce qu'ils représentent les composés du manganèse $(\eta^5\text{-}C_5H_5)Mn(CO)_2[P(C_6H_4\text{-m-}SO_3Na)_3]$, $(\eta^5\text{-}C_5H_5)Mn(CO)[P(C_6H_4\text{-m-}SO_3Na)_3]_2$.

9. Utilisation des composés selon une ou plusieurs des revendications 1 à 8 en tant que catalyseurs d'hydrogénation.

**10.** Utilisation des composés selon une ou plusieurs des revendications 1 à 8 en tant que catalyseurs pour le réglage de l'équilibre du gaz à l'eau.

**11.** Utilisation des composés selon une ou plusieurs des revendications 1 à 8 en tant que catalyseurs d'hydrocarbonylation.

**12.** Utilisation des composés selon une ou plusieurs des revendications 1 à 8 en tant que catalyseurs d'hydroformylation.

**13.** Utilisation des composés selon une ou plusieurs des revendications 1 à 8 en tant que catalyseurs d'oxydation.

**14.** Utilisation des composés selon une ou plusieurs des revendications 1 à 8 en tant que catalyseurs pour la condensation de doubles liaisons C-C avec des liaisons multiples C-C.

**15.** Mode de réalisation selon la revendication 14 caractérisé en ce que les composés sont utilisés en tant que catalyseurs pour la condensation allène/alcyne.

**16.** Utilisation des composés selon une ou plusieurs des revendications 1 à 8 en tant que catalyseurs pour la fixation par addition d'amines secondaires sur des doubles liaisons C-C.